# EUROPÄISCHE PATENTSCHRIFT

(11) **EP 1 949 955 B1**
(45) Veröffentlichungstag und Bekanntmachung des Hinweises auf die Patenterteilung: **21.07.2010**
(21) Anmeldenummer: 07033579.9
(22) Anmeldetag: 12.12.2007
(51) Int. Cl.: B01F 11/00, C12M 1/00

(54) **Objektlagervorrichtung mit Schüttelfunktion**
Storage device with shake function
Dispositif de stockage d'objets à fonction d'agitation

(30) Priorität: 26.01.2007 DE 102007004072
(43) Veröffentlichungstag der Anmeldung: 30.07.2008
(73) Patentinhaber: Thermo Electron LED GmbH, 63505 Langenselbold (DE)
(72) Erfinder: Kaufmann, Klaus, Dr., 81827 München (DE); Betz, Stefan, 63526 Erlensee (DE); Loscher, Helmut, 61130 Nidderau (DE); Heeg, Hubert, 63776 Mömbris (DE)
(74) Vertreter: Tomerius, Isabel

(56) Entgegenhaltungen:
- EP-A- 0 569 214
- EP-A- 1 443 101
- EP-A- 1 445 307
- EP-A- 1 721 964
- US-A- 4 669 225

## Beschreibung

Die Erfindung betrifft eine Objektlagervorrichtung mit Schüttelfunktion mit einer senkrecht angeordneten Lagerkassette, die mehrere übereinander angeordnete Objektlagerstellen aufweist, und mit einer über eine erste Antriebsverbindung mit der Lagerkassette funktional verbundenen Antriebseinrichtung, umfassend ein Antriebselement zum Durchführen einer Schüttelbewegung der Lagerkassette. Die Erfindung betrifft auch einen Klimaschrank mit einer vorgenannten Objektlagervorrichtung.

Unter einer Objektlagervorrichtung wird in diesem Zusammenhang eine Vorrichtung verstanden, in die Objekte an mehreren turmartig übereinander angeordneten Objektlagerstellen aufgenommen werden können. Mehrere im Sinne der Erfindung bedeutet dabei insbesondere mehr als fünf und ganz besonders mehr als zehn. Objekte sind dabei ganz besonders geschlossene Behältnisse, Mikrotiterplatten und ähnliche Behältnisse zur Aufnahme von Proben. Eine derartige Objektlagervorrichtungen ist beispielsweise aus der EP 1 155 743 A2 bekannt. Diese weist mehrere übereinander stapelartig angeordnete Einschübe zur Aufnahme von Probenträgern, wie insbesondere Mikrotiterplatten, auf. Die Integration einer Schüttelfunktion gestaltet sich hier allerdings schwierig, da bei dieser Objektlagervorrichtung die in die Einschübe eingebrachten Objekte in den Objektlagerstellen locker gelagert sind und somit durch eine Schüttelbewegung der Lagerkassette aus den Halterungen herausfallen könnten. Ferner sind Objektlagervorrichtungen mit Befestigungselementen für Objekte, wie beispielsweise die laschenartigen, über den Bodenbereich einer Objektlagerstelle vorstehenden Positionierelemente der EP 0 725 133 A2, bekannt. Diese ermöglichen aber nicht eine schüttelfeste Arretierung des Objektes, sondern dienen vielmehr ausschließlich der Gewährleistung eines vollständigen Einschiebens eines Objektes in eine Objektlagerstelle. Im Bereich von Schütteleinrichtungen ist die Verwendung von Haltewinkeln beispielsweise aus der EP 1 201 297 A1 bekannt. Dabei wird das Objekt von oben in die Haltewinkel eingestellt und locker durch die Haltewinkel in der in der Horizontalebene schüttelnden Schütteleinrichtung gehalten. Die Verwendung von Haltewinkeln scheidet jedoch für eine Objektlagereinrichtung mit mehreren übereinander angeordneten Objektlagerstellen aus, da dort beispielsweise ein von oben kommendes Einbringen der Objekte aufgrund des erhöhten Raumbedarfs nachteilig ist. Erste Verbesserungsansätze in diesem Zusammenhang gehen aus der DE 103 02 809 A1 hervor, die die Verwendung von ein- oder mehrteiligen Spannelementen in Form von Fixierwinkeln offenbart. Allerdings erfordert auch dieser Lösungsansatz das von oben kommende Einbringen des Objektes in die Objektlagerstelle, so dass diese Vorrichtung nur schlecht für die Lagerung und Schüttelung größerer Objektanzahlen bzw. größerer Lagerkassetten mit einer Vielzahl von übereinander liegenden Objektlagerstellen geeignet ist. Alternativ ist aus der EP 0 569 214 A2 eine Objektlagervorrichtung bekannt, die keine Lagerkassette mit mehreren übereinander angeordneten Objektlagerstellen, sondern mehreren übereinanderliegenden Schüttelebenen umfasst. Die jeweils zueinander benachbart liegenden Schüttelebenen werden phasenversetzt über entsprechend angeordnete exzentrisch gelagerte Antriebszahnräder angetrieben. Die Antriebszahnräder sind übereinanderliegend auf einer gemeinsamen Welle angeordnet, die von einem Motor in eine Rotationsbewegung versetzt wird. Im Gegensatz zur Objektlagervorrichtung der EP 0 569 214 A2 ist jedoch bei der Entwicklung von Objektlagervorrichtungen mit mehreren und insbesondere mehr als 10 Objektlagerstellen pro Kassette insbesondere die Gewährleistung einer gleichmäßigen Verteilung der Schüttelbewegung über die gesamte Länge der Lagerkassette hinweg problematisch. Ein ungleichmäßiges Ausmaß der Schüttelbewegung in den einzelnen Objektlagerstellen ist insofern besonders problematisch, als dass die erhaltenen Schüttelergebnisse nicht einheitlich über die Längsachse der Lagerkassette und somit nicht reproduzierbar sind. Die vorgenannten Nachteile treten besonders deutlich in Klimaschränken mit solchen Objektlagervorrichtungen, in denen mehrere Objekte übereinander gestapelt sind, hervor. Ein entsprechender Verbesserungsansatz geht aus der EP 1 721 964 hervor, die die Verwendung einer Gegengewicht-Anordnung zur Erzeugung einer höhenunabhängigen Schüttelamplitude offenbart.

Aufgabe der Erfindung ist somit, die Handhabung und den Betrieb einer Objektlagervorrichtung mit mehreren übereinander angeordneten Objektlagerstellen insbesondere im Hinblick auf eine gleichmäßige und reproduzierbare Schüttelung aller in einer Lagerkassette angeordneten Objekte zu verbessern.

Die Lösung der Aufgabe gelingt für eine Objektlagervorrichtung mit Schüttelfunktion mit einer senkrecht angeordneten Lagerkassette, die mehrere übereinander angeordnete Objektlagerstellen aufweist, und mit einer über eine erste Antriebsverbindung mit der Lagerkassette funktional verbundenen Antriebseinrichtung, umfassend ein Antriebselement zum Durchführen einer Schüttelbewegung der Lagerkassette, dadurch, dass sie eine weitere funktionale Verbindung der Antriebseinrichtung mit der Lagerkassette über eine zweite Antriebsverbindung aufweist, wobei die erste Antriebsverbindung mit einem Bodenbereich der Lagerkassette und die zweite Antriebsverbindung mit einem Kopfbereich der Lagerkassette verbunden ist, und dass eine Steuereinrichtung vorhanden ist, die zur Synchronisation der beiden Antriebsverbindungen ausgebildet ist. Ein wesentlicher Grundgedanke der Erfindung liegt somit darin, mehrere Antriebsverbindungen zur funktionalen Verbindung der Antriebseinrichtung mit der Lagerkassette in die Objektlagervorrichtung zu integrieren. Unter einer funktionalen Verbindung im Sinne der Erfindung ist dabei insbesondere eine Verbindung zur Übertragung von einer durch das Antriebselement, wie beispielsweise einem Motor, generierten Antriebsleitung zur Schüttelung auf die Lagerkassette zu verstehen. Dies kann über eine direkte Verbindung, beispielsweise über ein Getriebe, oder beispielsweise aber auch über magnetische Wechselwirkungen, die über die Antriebseinrichtung ausgelöst werden und im Bereich der Antriebsverbindung auf die Lagerkassette übertragen werden, geschehen. Entsprechend umfasst eine Antriebseinrichtung wenigstens ein Antriebselement und gegebenenfalls beispielsweise Getriebeelemente etc., die zur Übertragung der vom Antriebselement ausgeübten Antriebsleistung zur Auslösung der Schüttelbewegung der Lagerkassette dienen. Erfindungsgemäß sind die erste und die zweite Antriebsverbindung räumlich voneinander getrennt mit der Lagerkassette verbunden. Dazu ist es vorteilhaft, die Angriffspunkte der ersten und zweiten Antriebsverbindung möglichst weit voneinander zu beabstanden. Auf diese Weise ist es möglich, die Schüttelbewegung der Lagerkassette nicht nur einseitig, sondern mehrseitig und insbesondere zweiseitig auf die Lagerkassette zu übertragen. Somit gelingt die Ausführung einer besonders gleichmäßigen und über die gesamte Lagerkassette homogenen Schüttelbewegung. Ein weiterer wesentlicher Grundgedanke der Erfindung liegt in der Synchronisation der ersten und der zweiten Antriebsverbindung. Diese Synchronisation der Bewegungen der ersten und der zweiten Antriebsverbindung gewährleistet, dass die Lagerkassette über ihre gesamte Längsachse gleichmäßig geschüttelt wird und ermöglicht eine verwindungsfreie Schüttelbewegung der Lagerkassette. Mit einer erfindungsgemäßen Objektlagervorrichtung gelingt es somit besonders gut, reproduzierbare Schüttelergebnisse zu erzielen, so dass auch Lagerkassetten mit mehr als 10 und insbesondere mit mehr als 15 übereinander angeordneten Objektlagerstellen zuverlässig und gleichmäßig über den gesamten Lagerbereich und insbesondere über die gesamte Längserstreckung der Lagerkassette geschüttelt werden können.

Vorzugsweise liegen die erste und die zweite Antriebsverbindung relativ zur Lagerkassette einander gegenüber, wobei die erste Antriebsverbindung von unten und die zweite Antriebsverbindung von oben und besonders bevorzugt an sich gegenüberliegenden Randbereichen mit der wenigstens einen Lagerkassette funktional verbunden sind. Bevorzugt ist somit eine Verbindung der ersten Antriebsverbindung in Vertikalrichtung der Lagerkassette mit dem oben liegende Kopfbereich der Lagerkassette und der zweiten Antriebsverbindung mit dem unten liegende Bodenbereich der Lagerkassette. Eine derartige Verbindung der ersten und der zweiten Antriebsverbindung mit den Stirnseiten entlang der Längsachse der Lagerkassette ist besonders vorteilhaft, da auf diese Weise eine besonders gleichmäßige und über die gesamte Länge der Lagerkassette homogene Schüttelbewegung besonders gut gewährleistet werden kann.

In einer besonders bevorzugten Ausführungsform ist das Antriebselement ein elektromagnetischer Antrieb. Elektromagnetische Antriebe zeichnen sich durch ihre besonders hohe Funktionsverlässlichkeit und ihren vergleichsweise einfachen Aufbau aus.

Dabei ist die Antriebseinheit in einer besonders bevorzugten Ausführungsform zur Durchführung unterschiedlicher Schüttelbewegung und insbesondere zur Durchführung von Orbital-, Linear- und Diagonalschüttelbewegungen ausgebildet. Eine derartige Antriebseinheit ist vorteilhaft, da der Benutzer zwischen verschiedenen Schüttelbewegungen wählen kann. Vorzugsweise sind mehrere Schüttelbewegungen miteinander kombinierbar, so dass auf diese Weise besonders herausragende Schüttelergebnisse erhalten werden können.

Dabei ist es bevorzugt, wenn das Antriebselement diese unterschiedlichen Schüttelbewegungen direkt ausführen kann, da somit auf die Verwendung aufwendiger Getriebeelemente verzichtet werden kann. Typische Schüttelbewegungen der Erfindung betreffen Bewegungen der Lagerkassette in der XZ-Ebene bzw. in der Horizontalebene. Dabei handelt es sich beispielsweise um kreisförmige, innerhalb einer gradlinigen Strecke wechselnde, ellipsenförmige, bogenförmige oder gezackte Schüttelbewegungen. In Relation zu einer rechteckigen Bodenfläche einer Objektlagerstelle können die Bewegungsmuster somit beispielsweise linear oder auch diagonal erfolgen. Darüber hinaus können speziell mit einem Direktantrieb verschiedene Schüttelbewegungen miteinander kombiniert werden. Solche Schüttelbewegungen können besonders gut mit einem Antriebselement mit dem beispielsweise in der EP 1 201 297 A1 beschriebenen grundsätzlichen Wirkungsprinzip erhalten werden, bei dem unter Einwirkung von Magneten und Spulen eine Schüttelplatte direkt angetrieben wird. Durch dieses Zusammenspiel von Magneten und Spulen können in der Horizontalebene nahezu beliebige Bewegungsmuster durchgeführt werden. Außerdem erlaubt der magnetische Direktantrieb der EP 1 201 297 A1 eine Reduktion der bewegten Massen. Speziell dieses Wirkprinzip eines magnetischen Direktantriebs ist somit besonders vorteilhaft.

Vorzugsweise weist die Antriebseinrichtung ein erstes und ein zweites Antriebselement auf, wobei das erste Antriebselement über die erste Antriebsverbindung und das zweite Antriebselement über die zweite Antriebsverbindung mit der Lagerkassette verbunden ist, und wobei die Steuereinheit zur Synchronisation der durch das erste und das zweite Antriebselement durchgeführten Schüttelbewegung ausgebildet ist. Die Verwendung mehrerer Antriebselemente ermöglicht die Verwendung leistungsarmer Antriebselemente, wie beispielsweise leistungsarmer Elektromotoren. Dies ist insbesondere bei der Verwendung einer erfindungsgemäßen Objektlagervorrichtung in einem Klimaschrank und insbesondere in einem Inkubator von Vorteil, da leistungsarme Antriebe nur einen minimalen Wärmeeintrag bedingen. Auf diese Weise ist somit die Temperatursteuerung des Klimaschrankes bzw. des Inkubators erheblich erleichtert bzw. der durch die Antriebseinrichtung ausgelöste Temperaturfehler besonders gering, insbesondere soweit wesentliche Bauteile der Antriebseinrichtung unmittelbar in den temperierten Innenraum des Klimaschrankes bzw. des Inkubators integriert sind. Darüber hinaus lassen sich beispielsweise vergleichsweise hohe Drehzahlbereiche von bis zu 1500 Umdrehungen pro Minute bei einer Amplitude von 1 mm mit einer derartigen Antriebseinrichtung erreichen.

Besonders bewährt hat sich eine wenigstens teilweise feuchtigkeitsdichte Ausbildung der Antriebseinrichtung und insbesondere des ersten und des zweiten Antriebselementes der Antriebseinrichtung. Dies gilt speziell für diejenigen Teile der Antriebseinrichtung, die unmittelbar im temperierten Bereich, wie beispielsweise dem Innenraum eines Klimaschrankes und insbesondere dem Innenraum eines Inkubators, der erfindungsgemäßen Objektlagervorrichtung angeordnet sind. Diese besondere Ausführungsform erlaubt einerseits, die Objektlagervorrichtung nicht nur unter trockenen Klimabedingungen, sondern vielmehr auch unter Bedingungen mit hoher Luftfeuchtigkeit zu betreiben. Durch die feuchtigkeitsdichte Ausführung derjenigen Elemente der Antriebeinrichtung und insbesondere der ersten und zweiten Antriebselemente wird eine Beschädigung dieser Elemente durch kondensierendes Wasser verhindert. Andererseits bringt der Zugang zu diesen Bauteilen über den Innenraum der Objektlagervorrichtung für den Nutzer den Vorteil, dass diese Bauteile, beispielsweise zu Wartungszwecken, unmittelbar über den Innenraum erreichbar sind und nicht erst Gehäusebestandteile etc. entfernt werden müssen.

Es ist ferner vorteilhaft, wenn das erste und das zweite Antriebselement baugleich ausgeführt sind. Diese Ausführungsform ist insofern bevorzugt, als dass die Synchronisation der ausgeführten Schüttelbewegungen erheblich erleichtert ist. Zudem kann die Anzahl der zur Montage einer solchen Objektlagervorrichtung benötigten unterschiedlichen Teile im Herstellungsprozess reduziert werden. Eine solche Objektlagervorrichtung ist somit auch besonders preiswert in der Herstellung und Wartung.

Die Objektlagervorrichtung weist bevorzugt mehr als eine Lagerkassette, besonders zwei Lagerkassetten auf, die bevorzugt unabhängig voneinander geschüttelt werden können. Besonders bevorzugt ist eine Objektlagervorrichtung, die mindestens zwei Lagerkassetten aufweist, wobei jede der Lagerkassetten eine erste und eine zweite Antriebsverbindung aufweist. Auf diese Weise kann die Benutzungsflexibilität und gleichzeitig die Lagerkapazität der erfindungsgemäßen Objektlagervorrichtung gesteigert werden. Dabei sind die wenigstens zwei Lagerkassetten beispielsweise in der Horizontalebene nebeneinander oder auch übereinander angeordnet.

Besonders vielseitig nutzbar und damit vorteilhaft ist es ferner, dass die Steuereinheit zur voneinander getrennten Steuerung der zwei Lagerkassetten ausgebildet ist. Die Schüttelbewegungen der einzelnen Lagerkassetten lassen sich bei dieser Ausführungsform somit individuell über die Steuereinheit steuern. Dies ermöglicht beispielsweise das Anhalten der Schüttelbewegung einer Lagerkassette, wobei die zweite Lagerkassette weiterhin geschüttelt wird. Im Ergebnis ist diese Ausführungsform besonders benutzerfreundlich, da das Wechseln von Objekten in einer Lagerkassette möglich ist, ohne die Schüttelbewegung der anderen Lagerkassette/n zu unterbrechen, und gleichzeitig durch die verkürzten Unterbrechungsintervalle gleichmäßige und reproduzierbare Schüttelergebnisse erhalten werden können.

Um eine effektive Übertragung der Schüttelbewegung der Lagerkassette auf die in der Lagerkassette gelagerten Objekte zu gewährleisten, ist bei einer besonders bevorzugten Ausführungsform der Erfindung eine Feststelleinrichtung mit einem Feststellelement zum Feststellen des Objekts, insbesondere eines plattenförmigen Probenträgers wie beispielsweise einer Mikrotiterplatte, in einer der Objektlagerstellen und mit einer Stellvorrichtung vorhanden, wobei die Stellvorrichtung zum Verstellen des Feststellelementes zwischen einer "Feststell"-Position und einer "Freigabe"-Position ausgebildet ist, und wobei das Objekt in der "Feststell"-Position in einer der Objektlagerstellen festgestellt ist und in der "Freigabe"-Position aus dieser Objektlagerstelle entnehmbar ist. Ein Feststellelement im Sinne der Erfindung ist somit wenigstens ein Bauteil, welches ein Objekt in einer Objektlagerstelle feststellen kann, so dass das Objekt in der Objektlagerstelle insbesondere im Verlauf einer auf die Lagerkassette ausgeübten Schüttelbewegung nicht hin- und herrutscht. Diese Feststellung betrifft insbesondere die Feststellung des Objektes in der Objektlagerstelle im Hinblick auf Horizontalbewegungen der Lagerkassette, wie sie im Vorhergehenden bereits beschrieben wurden. Die erfindungsgemäße Stellvorrichtung ermöglicht nun ein von dem Feststellelement in der Objektlagerstelle festgestelltes Objekt insofern zu lösen, als dass das Objekt nach dem Lösen der Feststellung aus der Objektlagerstelle herausgenommen werden kann. Dies geschieht beispielsweise durch ein Herausziehen des Objektes aus der einschubartigen Objektlagerstelle. Ein wesentlicher Grundgedanke der Erfindung liegt somit in der aktiven Kontrolle der Positionierung des Feststellelementes durch die Stellvorrichtung. Dies ist insofern besonders vorteilhaft, da ein Lösen des Feststellelementes nicht von dem Objekt selber, wie es beispielsweise bei den Haltewinkeln aus dem Stand der Technik der Fall ist, gesteuert wird. Durch die aktive Umstellung des Feststellelementes von der "Feststell"-Position in die "Freigabe"-Position durch die Stellvorrichtung ist vielmehr eine objektunabhängig ablaufende Umstellung des Feststellelementes bzw. ein Lösen und Feststellen des Feststellmechanismus ohne eine Bewegung des Objekts in der Objektlagerstelle möglich. Die Erfindung vereint somit gleich mehrere Vorteile. Einerseits ist das Objekt in der Objektlagerstelle speziell während einer Schüttelung der Lagerkassette in Relation zur Lagerkassette ortsfest und stabil und insbesondere in der vorzugsweise horizontalen Bewegungsebene festgestellt. Andererseits ist ein einfaches und insbesondere ruckelfreies Einbringen und Herausnehmen eines Objekts aus dem Objektlagerraum möglich, da der Feststellmechanismus aktiv und unabhängig vom Objekt gesteuert werden kann.

Vorzugsweise sind über die Stellvorrichtung Feststellelemente in mehreren Objektlagerstellen und insbesondere in allen Objektlagerstellen einer Lagerkassette kombiniert von der "Feststell"-Position in die "Freigabe"-Position stellbar. Durch die eine Stellvorrichtung lassen sich somit mehrere und vorzugsweise alle Feststellelemente einer Lagerkassette zeitgleich und gemeinsam von der "Feststell"-Position in die "Freigabe"-Position schalten. Der Benutzer hat auf diese Weise die Möglichkeit, alle in einer Lagerkassette durch Feststellelemente festgestellten Objekte in einem Arbeitsschritt von der Feststellung zu lösen. Diese Ausführungsform ist insofern besonders effizient, da ein einzelnes und zeitraubendes Lösen jedes einzelnen Feststellelementes einer Lagerkassette entfällt.

In einer besonders vorteilhaften Ausführungsform weist die Stellvorrichtung eine vertikal in der Objektlagervorrichtung angeordnete Stellleiste zur kombinierten Schaltung aller Feststellelemente der wenigstens einen Lagerkassette zwischen der "Feststell"-Position und der "Freigabe"-Position auf. Über eine Positionsänderung der vorzugsweise schwenkbar gelagerten Stellleiste erfolgt bei dieser erfindungsgemäßen Ausführungsform somit die Steuerung des Umschaltens aller Feststellelemente zur Freigabe der in den einzelnen Objektlagerstelle festgestellten Objekte. Dabei hat sich eine Steuerung über eine Stellleiste als besonders zuverlässig herausgestellt.

Vorzugsweise weist das Feststellelement der Feststelleinrichtung einen zweiarmigen Hebel mit einem Steuerhebel, der zur Schaltung des Feststellelementes zwischen der "Feststell"-Position und der "Freigabe"-Position mit der Stellvorrichtung und insbesondere mit einer Stellleiste in Kontakt steht, und einen Feststellhebel, der zur Feststellung des Objekts in der Objektlagerstelle zumindest mit einem Teilbereich seitlich gegen das Objekt drückt, auf. Die Feststelleinrichtung weist somit eine Hebelfunktion auf, über die die Funktion des Feststellmechanismus des Feststellelementes gesteuert wird. Der Steuerhebel dient dabei als Steuerkontakt, insbesondere in Wirkverbindung mit einer Stellleiste, so dass über den Steuerhebel die Positionierung des zweiarmigen Hebels von der Stellvorrichtung gesteuert wird. Bei der Stellleiste handelt es sich im Rahmen der Erfindung insbesondere um ein Bauteil, welches leistenartig ausgebildet ist und ganz besonders die gleichzeitige Ansteuerung mehrerer Steuerhebel mehrerer Objektlagerstellen ermöglicht. Dazu verläuft die Stellleiste beispielsweise im Wesentlichen vertikal entlang der Lagerkassette. Der Feststellhebel dient zur unmittelbaren Feststellung des Objektes in der Objektlagerstelle. Dazu legt sich der Feststellhebel üblicherweise in der "Feststell"-Position seitlich an das Objekt, wie beispielsweise eine Mikrotiterplatte, an und drückt dieses seitlich gegen einen der Kontaktseite zwischen Objekt und Feststellhebel gegenüberliegenden Wandbereich der Objektlagerstelle. Ferner können Beschichtungen und/oder Druckelemente zwischen dem Feststellhebel und dem Objekt vorhanden sein, die letztendlich die seitliche Feststellwirkung des Feststellhebels in der "Feststell"-Position auf das Objekt übertragen. Derartige Beschichtungen und/oder Druckelemente können die Positionsstabilität des festgestellten Objektes zusätzlich verbessern. Der Feststellhebel kann somit mittelbar, aber auch unmittelbar auf das festzustellende Objekt wirken.

Dabei hat sich die Ausbildung des zweiarmigen Hebels als Blattfeder besonders bewährt. Blattfedern weisen einen einfachen Aufbau und eine hohe Funktionszuverlässigkeit auf und sind zudem vergleichsweise preiswert in der Herstellung. Blattfedern sind somit vorteilhaft.

Besonders effektiv und damit vorteilhaft ist eine Ausbildung des Feststellhebels in der "Feststell"-Position in der Weise, das dieser ein in die Objektlagerstelle eingebrachtes Objekt im Bereich der Objektvorderkante zur Verhinderung eines Herausrutschens des Objektes aus der Objektlagerstelle umgreift. Zusätzlich zu der seitlichen Feststellwirkung des Feststellhebels quer und insbesondere senkrecht zur Einschubrichtung des Objektes in die Objektlagerstelle ist somit in dieser bevorzugten Ausführungsform eine zusätzliche Rutschsicherung vorgesehen, die ein Herausrutschen des Objektes insbesondere entgegen der Einschubrichtung des Objektes verhindert. Dazu umgreift der Feststellhebel wenigstens in einem Teilbereich vorzugsweise die Objektvorderkante. Objektvorderkante bezieht sich in diesem Zusammenhang auf die Kante des Objektes und insbesondere der Mikrotiterplatte auf der Einschubseite der Lagerkassette. Vorzugsweise ist diese Rutschsicherung derart ausgestaltet, dass durch das wenigstens teilweise Umgreifen der Objektvorderkante durch den Feststellhebel eine in Einschubrichtung des Objektes in die Objektlagerstelle wirkende Kraft auf das Objekt ausgeübt wird. Diese Ausführungsform ist hinsichtlich der Stabilität der Lagerung des Objektes in der Objektlagerstelle besonders zuverlässig, da das Objekt nicht nur seitlich eingeklemmt, sondern zudem vor einem unerwünschten Herausrutschen aus der Objektlagerstelle gesichert ist. Vorzugsweise sind die Rutschsicherung und der Feststellhebel einteilig ausgebildet, so dass der Feststellhebel in "Feststell"-Position sowohl eine seitliche als auch eine in Einschubrichtung des Objektes wirkende Kraft auf das Objekt ausübt.

Vorzugsweise ist der Feststellhebel in der Weise federbeaufschlagt, dass die Federbeaufschlagung in die "Feststell"-Position des Feststellhebels wirkt. Eine derartige Federbeaufschlagung bewirkt somit, dass durch ein Lösen der Stellvorrichtung der Feststellhebel durch die Federbeaufschlagung, die beispielsweise durch eine Druckfeder erreicht wird, unabhängig von der Stellvorrichtung wieder in die "Feststell"-Position verfährt. Ein aktives Umstellen, beispielsweise durch die Stellleiste, des Feststellhebels von der "Freigabe"-Position in die "Feststell"-Position durch die Stellvorrichtung ist somit nicht erforderlich. Diese Ausführungsform ist vorteilhaft, da die Stellvorrichtung nicht zur Rückstellung des Feststellhebels ausgebildet sein muss und somit eine vereinfachte Bauweise aufweisen kann.

Zur Lösung der Aufgabe hat sich besonders eine motorgetriebene Feststelleinrichtung bewährt. Die motorgetriebene Feststelleinrichtung ist insofern von Vorteil, als dass diese besonders benutzerfreundlich ist. Das manuelle und je nach Größe der Objektlagervorrichtung kraftaufwendige Umlegen der Stellvorrichtung entfällt hier. Dabei hat es sich als besonders vorteilhaft herausgestellt, speziell die Stellvorrichtung und insbesondere die Stellleiste der Stellvorrichtung der Feststelleinrichtung motorgetrieben auszustatten. Insbesondere in Kombination mit der vorgenannten Federbeaufschlagung ergibt sich auf diese Weise eine besonders benutzerfreundliche Objektlagervorrichtung, da sowohl die durch die Federbeaufschlagung ausgelöste Feststellung, als auch die durch die motorgetriebene Stellvorrichtung ausgelöste Freigabe der Objekte automatisch abläuft und keinen manuellen Einsatz erfordert.

Es ist ferner von Vorteil, wenn die Feststelleinrichtung ein selbsthemmendes Getriebe aufweist. Durch die Verwendung eines selbsthemmenden Getriebes ist es möglich, die Positionierung der Feststelleinrichtung und insbesondere der Stellvorrichtung auf einfache Art und Weise aufrechtzuerhalten, ohne dass beispielsweise eine Dauerbestromung eines Antriebs notwendig ist.

Die Kombination einer erfindungsgemäßen Objektlagervorrichtung mit der vorstehend dargestellten Feststelleinrichtung stellt somit eine besonders elegante Lösung der Aufgabe dar. Mit dieser Ausführungsform lassen sich nämlich die Vorteile der vorgenannten Feststelleinrichtung mit den Vorteilen der erfindungsgemäßen Antriebsvorrichtung vereinen. Eine solche Objektlagervorrichtung ermöglicht ganz besonders gut die gleichzeitige Sicherstellung reproduzierbarer und gleichmäßiger Schüttelbewegungen über die gesamte Längsachse der senkrecht angeordneten Lagerkassette.

Besonders bewährt hat sich der Einsatz einer der vorstehend aufgeführten erfindungsgemäßen Objektlagervorrichtungen in einem Klimaschrank und insbesondere einem Inkubator. So ist es ja beispielsweise möglich, durch die spezielle Antriebsvorrichtung leistungsarme Motoren mit den oben bereits erwähnten Vorteilen zu verwenden. Und auch die oben genannten erfindungsgemäßen Objektlagervorrichtungen mit Feststelleinrichtung eignet sich beispielsweise aufgrund der einfachen Bedienbarkeit und der hohen Funktionszuverlässigkeit besonders gut für einen Klimaschrank und ganz besonders für einen Inkubator und hier insbesondere einen automatisierten Inkubator, da eine automatische Feststell- und Freigabeeinrichtung besonders vorteilhaft mit einer automatisierten Be- und Entladung der gelagerten Objekte kombinierbar ist.

In diesem Zusammenhang ist auch eine andere Weiterbildung der Erfindung zu nennen, die besonders ein automatisiertes Be- und Entladen der Objekte erleichtert. In dieser Weiterbildung ist eine Arretiervorrichtung vorgesehen, welche die Lagerkassette in einer definierten Position arretiert, wenn sich das wenigstens eine Feststellelement in der "Freigabe"-Position befindet und somit die gelagerten Objekte entnommen oder Objekte eingelagert werden können. Um geschüttelt werden zu können, muss die Lagerkassette mit einem gewissen Spiel gelagert sein. Würde der Schüttelvorgang einfach unterbrochen, um Objekte zu entnehmen oder einzulagern, wäre die Position der Lagerkassette also je nach Zeitpunkt des Stopps des Schüttelvorgangs unterschiedlich. Ein positionsgenaues automatisches Beschicken und Entnehmen von Objekten aus der Lagerkassette wäre also kaum möglich. Erfindungsgemäß ist daher eine Arretiervorrichtung vorgesehen, die die Lagerkassette bei Freigabe der Feststellelemente in eine definierte Be- und Entladeposition bringt und dort arretiert. Um ein unbeabsichtigtes Verrutschen der Objekte zu vermeiden, wird die Lagerkassette zweckmäßig zuerst in die Be- und Entladeposition gebracht, bevor die Objekte durch Umschalten in die "Freigabe"-Position freigegeben werden. Nach dem Umschalten der Feststellelemente in die "Feststell"-Position wird die Arretierung der Lagerkassette dann wieder gelöst, sodass erneut ein Schüttelvorgang durchgeführt werden kann. Alle Vorgänge werden zweckmäßig von der Steuereinheit gesteuert.

Nachfolgend wird die Erfindung anhand der in den Figuren dargestellten Ausführungsbeispiele weiter erläutert. Es zeigen schematisch:
- Figur 1: schematische maßstabsgerechte Explosionsdarstellung einer Objektlagervorrichtung;
- Figur 2: perspektivische Darstellung einer Stellvorrichtung mit Feststellelementen;
- Figur 3a: schematische Draufsicht auf eine Stellvorrichtung mit Feststellelementen in "Feststell"-Position;
- Figur 3b: Ausschnittsansicht eines Horizontalschnitts entlang eines Feststellelements in "Feststell-Position;
- Figur 4a: schematische Draufsicht auf eine Stellvorrichtung mit Feststellelementen in "Freigabe"-Position;
- Figur 4b: Ausschnittsansicht eines Horizontalschnitts entlang eines Feststellelementes in "Freigabe-Position; und
- Figur 5: Ausschnittsansicht eines Horizontalschnitts entlang einer Blattfederbefestigung zwischen zwei Feststellelementen in "Feststell-Position.

Bei den im Folgenden dargestellten Ausführungsformen sind gleiche Bestandteile mit gleichen Bezeichnungen versehen.

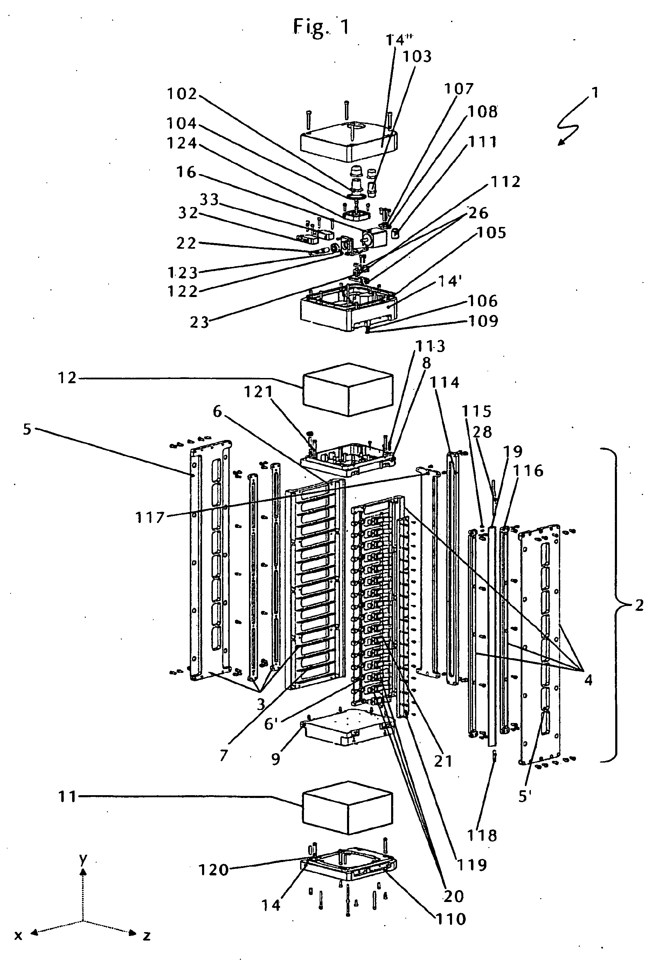

Die Objektlagervorrichtung 1 gemäß Fig. 1 umfasst eine Lagerkassette 2 mit zwei vertikal verlaufenden Seitenwänden 3 und 4, die unter anderem jeweils eine Stütze 5 bzw. 5' und eine leiterartige Seitenwandplatte 6 bzw. 6' aufweisen. An den leiterartigen Seitenwandplatten 6 bzw. 6' der Seitenwände 3 und 4 sind paarweise sich gegenüberliegende schienenartige Führungen 7 (in Fig. 1 allein auszugsweise für die linke Seitenwand 3 angegeben) angeordnet, die zum Einschub und Führen von Objekten (nicht dargestellt) und insbesondere Mikrotiterplatten in der Lagerkassette 2 ausgebildet sind. Ein Führungspaar (d.h. jeweils eine schienenartige Führung der Leiter 6 und eine auf gleicher Höhe angeordnete schienenartige Führung der Leiter 6' (in Fig. 1 nicht sichtbar) legt somit jeweils eine Objektlagerstelle der Objektlagervorrichtung 1 fest. Oberhalb und unterhalb der Seitenwände 3 und 4 ist jeweils eine Schwingplatte 8 bzw. 9 angeordnet, die die senkrecht angeordnete Lagerkassette 2 an den Stirnseiten nach oben (obere Schwingplatte 8) bzw. nach unten (untere Schwingplatte 9) begrenzt. An die Schwingplatte 8 bzw. 9 schließt sich jeweils auf der der Lagerkassette in Vertikalrichtung (y-Achse in Fig. 1) gegenüberliegenden Seite ein Antriebselement (Antriebselement 11 unterhalb der Lagerkassette 2 und Antriebselement 12 oberhalb der Lagerkassette 2) an, die durch eine Steuereinheit (nicht dargestellt) synchronisiert die Schüttelbewegung der Lagerkassette 2 in der Horizontalebene (XY-Ebene in Fig. 1) antreiben bzw. mit jeweils einer der beiden Schwingplatten 8 oder 9 in funktionaler Verbindung stehen. Die Antriebselemente 11 und 12 sind durch die beiden Boxen in Fig. 1 schematisch angedeutet. Die Synchronisation der beiden Antriebselemente 11 und 12 durch die Steuereinheit ermöglicht eine verwindungsfreie und gleichmäßige Schüttelung der senkrecht angeordneten Lagerkassette 2 in der Horizontaleben. Diese gleichmäßige Schüttelung gelingt dabei über die Längsachse der gesamten Lagerkassette 2 hinweg. Bei den Antriebselemente 11 und 12 handelt es sich um elektromagnetische Direktantriebe, deren grundsätzliche Ausbildung beispielsweise in der EP 1 201 297 A1 beschrieben ist, umfassend Magnete und Spulen, wobei die Bewegung durch eine Zusammenwirken der Magnete und Spulen hervorgerufen wird. Die Antriebselemente 11 und 12 sind auf der der Lagerkassette 2 in Vertikalrichtung abgewandten Seite an ein Gehäuse eines Klimaschrankes angeschlossen (in Fig. 1 sind allein die Verbindungsstellen zu diesem Gehäuse dargestellt, das Gehäuse an sich ist in Fig. 1 nicht dargestellt). Von dem Gehäuse sind in Fig. 1 lediglich die Teile 14, 14' und 14" dargestellt, wobei das Gehäuse dei Objektlagervorrichtung 1 bei der in Fig. 1 dargestellten Ausführungsform die Lagerkassette 2 erfindungsgemäß nahezu vollständig umgibt und einen mit einer Tür verschließbaren Durchtrittsbereich (nicht dargestellt) umfasst, durch den Objekte vom Inneren des Gehäuses nach außen und umgekehrt, beispielsweise mittels eines Bestückungsroboters, transportiert werden können. Mit dem in Fig. 1 dargestellten Objektlagervorrichtung 1 in einem Klimaschrank (Gehäuse nicht dargestellt) ist somit letztendlich eine Bewegung der in der Lagerkassette 2 gelagerten Objekte unter kontrollierten Klimabedingungen möglich. Dazu sind insbesondere die beiden Antriebselemente 11 und 12 feuchtigkeitsdicht ausgebildet, womit eine Bewegung von in der Objektlagervorrichtung 1 gelagerten Objekten (nicht dargestellt) auch unter feuchten Bedingungen möglich ist, ohne dass beispielsweise kondensierendes Wasser in die unmittelbar im klimatisierten Innenraum der Klimaschrankes (nicht dargestellt) angeordneten Antriebselemente 11 und 12 eindringen kann.

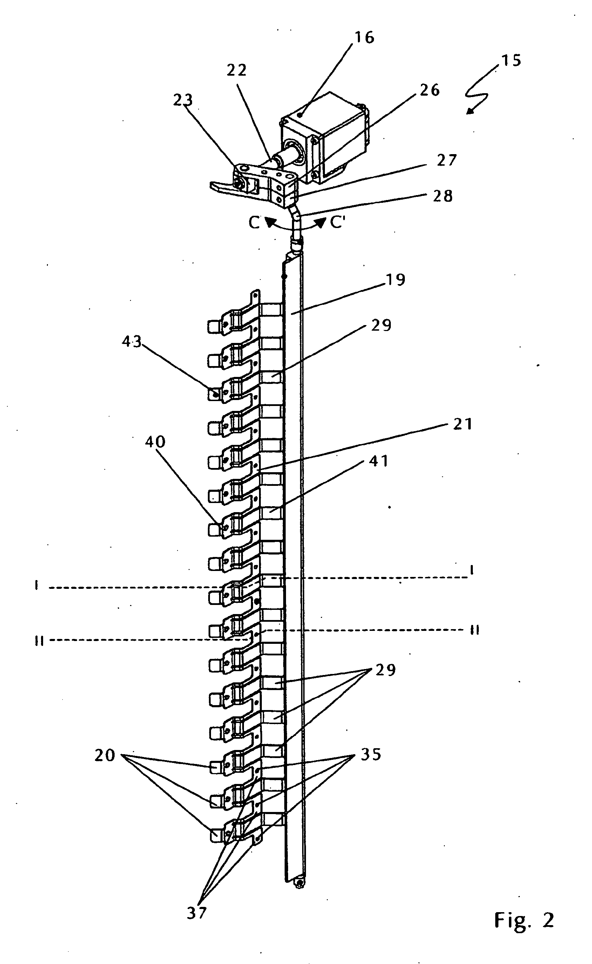

Die Objektlagervorrichtung 1 gemäß Fig. 1 weist ferner eine Feststellvorrichtung 15 (im Einzelnen in Fig. 2 dargestellt), umfassend insbesondere einen Antriebsmotor 16, zwei Mikroschalter 32 und 33, ein Getriebe mit unter anderem einer Gewindespindel 22, einer Mitnehmermutter 23, einem gabelartigem Übertragungselement 26 und einer Schwenkachse 28, eine Stellleiste 19 (Exzenterleiste) und eine Vielzahl blattfederartiger Feststellelemente 20 (in Fig. 1 sind der Übersichtlichkeit halber nur eine Auswahl der blattfederartigen Feststellelemente 20 ausdrücklich bezeichnet), auf. Die einzelnen blattfederartigen Feststellelemente 20 sind miteinander verbunden und bilden in ihrer Gesamtheit eine Federleiste 21. Der Antriebsmotor 16 der Feststellvorrichtung 15 ist oberhalb der Lagerkassette 2 angeordnet und weist gemäß Fig. 2 eine Gewindespindel 22 (das Gewinde selbst ist in den Figuren nicht dargestellt) auf, durch deren Drehung eine Mitnehmermutter 23 mit Innengewinde (nicht dargestellt) entlang der Gewindespindel 22 in Relation zur Gewindespindel 22 linear verfahrbar ist.

Weitere in der Fig. 1 näher bezeichnete Bauteile der Objektlagervorrichtung sind die Kabeldurchführungen 102 und 103, O-Ringe 104 bis 106, die Scheibe 107, die Platte 108, Buchsen 109 und 110, der Zapfen 111, der Gewindestift 112, der Passstift 113, die hintere Einschubbegrenzung 114, der Gewindestift 115, Stützen 116 und 117 der Schwingplatten, der Stift 118, die Druckleiste 119, die Stützen 120 und 121, der Motorträger 122, das Kugellager 123 und die Adapterplatte 124.

Die perspektivische Darstellung in Fig. 2 betrifft wesentliche Bestandteile der Feststellvorrichtung 15 aus Figur 1 und verdeutlicht die räumliche Anordnung des Antriebsmotors 16 der Feststellvorrichtung 15 in Relation zur Stellleiste 19 und den blattfederartigen Feststellelementen 20 (der Übersichtlichkeit halber sind in Fig. 2 von allen in der Stellleiste 19 vorhandenen Feststellelementen 20 nur die drei unteren Feststellelemente 20 ausdrücklich bezeichnet) bzw. der Federleiste 21. Die einzelnen blattfederartigen Feststellelemente 20 der Federleiste 21 sind jeweils zur Feststellung eines Objektes (in den Figuren 1 und 2 nicht dargestellt) in jeweils einer Objektlagerstelle vorgesehen. Dabei sind die einzelnen Blattfedern bzw. blattfederartigen Feststellelemente 20 gemäß Fig. 2 miteinander verbunden und bilden in ihrer Gesamtheit die Federleiste 21. Konkret sind die einzelnen blattfederartigen Feststellelemente 20 in ihrer Gesamtheit kammartig zueinander angeordnet, wobei jeweils ein Kammzinken bzw. ein Finger (= jeweils ein blattfederartiges Feststellelement 20) für die Feststellung eines Objektes in einer Objektlagerstelle vorgesehen ist.

Gemäß Fig. 2 treibt der Antriebsmotor (16) eine Drehbewegung der Gewindespindel 22 an, über deren Gewindeverlauf (nicht dargestellt) die Mitnehmermutter 23 geführt wird, die in einem gabelartigen Übertragungselement 26 drehbar gelagert ist. Dem gabelartigen Bereich des Übertragungselements 26 gegenüberliegend weist das Übertragungselement 26 einen Verbindungsbereich 27 zu einer Schwenkachse 28 auf, die mit der Stellleiste 19 (Exzenterleiste) verbunden ist. Durch eine durch den Antriebsmotor 16 angetriebene Drehbewegung der Gewindespindel 22 um ihre Längsachse verfährt die Mitnehmermutter 23 mit Innengewinde somit entlang der Rotationsachse der Gewindespindel 22 mit Außengewinde. Die Linearbewegung der Mitnehmermutter 23 bewirkt wiederum über das Übertragungselement 26 eine Schwenkbewegung um die Schwenkachse der Stellleiste 19. Diese Schwenkbewegung ist durch den Pfeil C-C' in Fig. 2 angedeutet. Erfolgt die Verschwenkung der Stellleiste 19 zu den blattfederartigen Feststellelementen 20 der Federleiste 21 hin (Pfeilrichtung C), schlägt die Stellleiste 19 zunächst gegen einen fingerartigen Anschlagsbereich 29 der Federleiste 21 an. Wird die Stellleiste 19 weiter in diese Richtung geschwenkt, werden letztendlich die blattfederartigen Feststellelemente 20 durch die Stellleiste 19 verschwenkt. Die Stellleiste 19 übt somit eine Stellkraft auf die blattfederartigen Feststellelemente 20 aus. Die Stellleiste 19 ist dazu in ihrem Fußbereich (in Fig. 2 unten) in der Lagerkassette (in Fig. 2 nicht weiter dargestellt) schwenkbar gelagert.

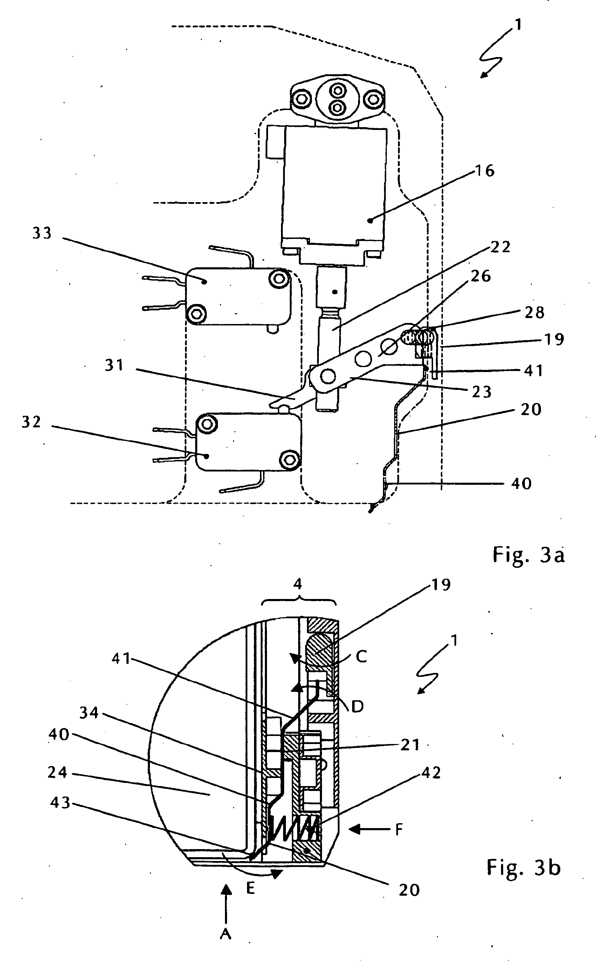

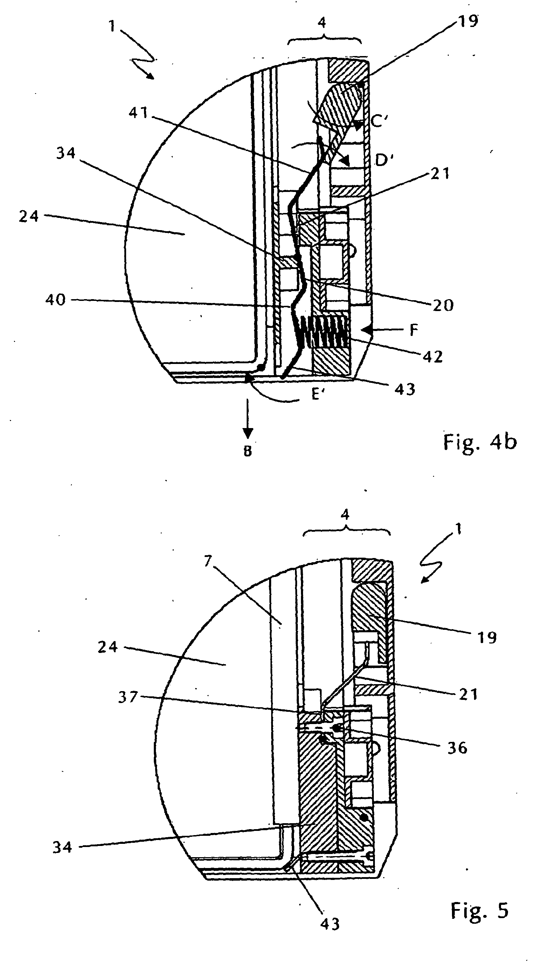

Ein weiterer wesentlicher Bestandteil der Feststellvorrichtung 15 ist die bereits vorstehend erwähnte kammartig ausgebildete Federleiste 21, die jeweils ein blattfederartiges Feststellelement 20 pro Objektlagerstelle umfasst. Diese ist gemäß Fig. 1 an einer Seitenwand der Lagerkassette (in Fig. 1 an der Leiter 6' der Seitenwand 4) mittels der Befestigungsschrauben 36 (in Fig. 5 ist exemplarisch allein eine der Befestigungsschrauben 36 bezeichnet) angebracht. Jedes Feststellelement umfasst einen Steuerhebel 41, der durch die Stellleiste 19 im Anschlagsbereich 29 kontaktiert werden kann, und einen Feststellhebel 40, der zum Feststellen eines in einer Objektlagerstelle gelagerten Objektes (in Fig. 2 nicht dargestellt) ausgebildet ist. Auch dies ist in Fig. 2 nur beispielhaft an einem Feststellelement 20 näher bezeichnet. Die Befestigungsschrauben 36 werden durch die Federleiste 21 in dem Bereich zwischen dem Feststellhebel 40 und dem Steuerhebel 41 durch eine Durchgangsöffnung 35 geführt und legen somit den Drehpunkt der blattfederartigen Feststellelement 20 fest. Durch die Schwenkbewegung der Stellleiste 19 kann diese die Federleiste 21 kontaktieren, womit im Ergebnis die Stellung aller blattfederartigen Feststellelemente 20 der Lagerkassette gemeinsam von einer "Feststell"-Position, in der ein in einer Objektlagerstelle befindliches Objekt durch das Feststellelement in seiner Position festgestellt wird, in eine "Freigabe"-Position, in der die Fixierung des in der Objektlagerstelle befindlichen Objekts durch das Feststellelement aufgehoben ist, stellbar ist. Die detaillierte Funktionsweise der Feststellvorrichtung 15 aus Fig. 2 ist in den Figuren 3a bis 5 näher veranschaulicht.

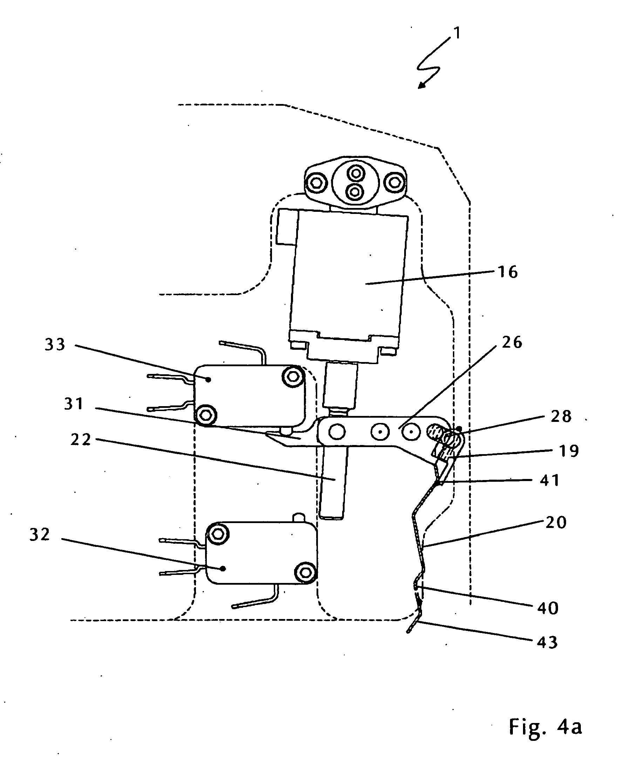

Die Figuren 3a und 3b betreffen die Feststellvorrichtung 15 in der "Feststell"-Position, in der ein Objekt 24 (in den Fig. und 4b ist nur ein Teilbereich des Objekts 24 dargestellt), im vorliegenden Fall eine Titerplatte, in einer Objektlagerstelle festgestellt ist. Die Einschubrichtung des Objekts 24 in die Objektlagerstelle der Objektlagervorrichtung 1 ist durch den Pfeil A angegeben. Die "Freigabe"-Position geht aus den Figuren 4a und 4b hervor, in denen das Objekt 24 (in Fig. 4b ebenfalls nur teilweise dargestellt) aus der Objektlagerstelle in Richtung des Pfeiles B herausziehbar ist. In den Figuren 3a und 4a sind ferner einige Wandverläufe der Objektlagervorrichtung mit gestrichelten Linien angedeutet. Bezüglich der in eine Objektlagervorrichtung 1 installierten Feststellvorrichtung 15 aus Fig. 2 repräsentieren die Figuren 3b und 4b einen Horizontalschnitt durch die Feststellvorrichtung entlang der Linie I-I aus Fig. 2 und die Fig. 5 einen Horizontalschnitt entlang der Linie II-II aus Fig. 2.

Die Figuren 3a und 4a betreffen eine Draufsicht von oben auf einen Teilbereich der Lagerkassette 2 der Objektlagervorrichtung 1 mit der Feststellvorrichtung 15 aus Fig. 2. Gemäß der Figuren 3a und 4a weist das Übertragungselement 26, welches die von der Gewindespindel 22 durchlaufene Mitnehmermutter 23 in der Bewegungsebene der Lagerkassette 2 schwenkbar lagert und über die Schwenkachse 28 mit der Stellleiste 19 in Verbindung steht, einen Kontaktfinger 31 zur Schalterbetätigung der sich gegenüberliegend angeordneten Schalter 32 und 33 auf. Über die Schalter 32 und 33 erfolgt die Steuerung des Betriebsintervalls des Antriebsmotors 16, so dass das Ausmaß der Schwenkbewegung der Stellleiste 19 gemäß der Pfeilrichtungen C bzw. C' (gemäß der Fig. 2, 3b und 4b) von der Kontaktierung der jeweiligen Schalter 32 und 33 abhängt. Die durch den Antriebsmotor 16 angetriebene Schwenkbewegung der Stellleiste 19 verläuft in Richtung C verläuft somit bis zur Betätigung des Schalters 33 durch den Kontaktfinger 31 und in Richtung C' bis zur Betätigung des Schalters 32.

In der "Feststell"-Position gemäß der Figuren 3a und 3b wirkt das blattfederartige Feststellelement 20 bezüglich der Einschubrichtung A orthogonal bzw. seitlich gegen das in die Objektlagerstelle eingebrachte Objekt 24 und drückt dieses gegen den dem Feststellelement 20 gegenüberliegenden Seitenwandbereich (nicht dargestellt) der Objektlagerstelle. Diese Wirkung des Feststellelementes 20 wird durch eine Druckfeder 42, die auf der objektabgewandten Seite des Feststellelementes 20 zwischen einem Wandbereich der Seitenwand 4 und dem Feststellelement 20 angeordnet ist, hervorgerufen. Die Druckfeder 42 wirkt in Richtung des Pfeiles F (Fig. 3b und 4b) auf das Feststellelement 20. Im Rahmen der Erfindung ist es dabei auch möglich, nicht für jede Objektlagerstelle eine einzelnen Druckfeder 42 vorzusehen. In der Ausführungsform gemäß der Figuren 1 bis 5 ist allerdings für jede Objektlagerstelle bzw. für jedes Feststellelement 20 jeweils eine Druckfeder 42 in der Objektlagervorrichtung angeordnet (in der Schnittansicht aus den Fig. 3b und 4b ist allerdings jeweils nur eine Druckfeder 42 dargestellt). Auf der dem Druckfederanschlagsbereich, in dem die Federleiste 21 in einem Teilbereich parallel zur Seitenwand 4 der Objektlagerstelle ausgebildet ist, am Feststellelement 20 gegenüberliegenden Seite der Federleiste 21 ist eine Zwischenwand 34 vorhanden, die diesen Bereich der Federleiste 21 und den das Objekt 24 beherbergenden Innenraum der Lagerkassette 2 räumlich trennt. In der "Feststell"-Position schlägt das Feststellelement 20 der Federleiste 21 mit diesem Teilbereich gegen diese Zwischenwand 34 an, womit das Ausmaß der Bewegung des Feststellelementes 20 in Feststellrichtung E' (Fig. 4b) begrenzt wird. Das Feststellelement 20 weist zur Einschuböffnung der Objektlagerstelle hin (in der Ausschnittsdarstellung gemäß Figur 3b unten) ferner einen Umgriff 43 auf, der die dem Objektlagerstellenausgang zugewandten Vorderkante des Objekts 24 (im vorliegenden Fall somit der Titerplatte) umgreift und diese durch diesen Umgriff neben der Feststellung in der Objektlagerstelle gleichzeitig in Einschubrichtung A in die Objektlagerstelle hineindrückt. Im Spitzenbereich bzw. im Bereich des Umgriffs 43 ragt das Feststellelement 20 in der "Feststell"-Position somit in den Innenraum der Objektlagerstelle hinein. Das Feststellelement 20 bzw. die Federleiste 21 ist ferner in der Weise durch die Seitenwand 4 der Lagerkassette geführt, dass es an dem zum Umgriff 43 abgewandten Teil des Feststellelementes 20 mit einem Kontaktbereich in den Schwenkraum der Stellleiste 19 hineinragt.

Die Federleiste 21 ist an der Zwischenwand 34 in der Horizontalebene schwenkbar gelagert. Eine solche Lagerung kann aufgrund der Federeigenschaften der Federleiste beispielsweise mit Schrauben oder Bolzen erhalten werden, die die Blattfeder mit der Zwischenwand als Teil der Seitenwand verbinden. Im vorliegenden Ausführungsbeispiel weist die Federleiste 21 dazu einen Befestigungsbereich 37 auf, in dem jeweils zwischen zwei Objektlagerstellen eine Befestigungsschraube 36 durch eine Durchgangsöffnung 35 der Federleiste 21 geführt ist. Eine solche Verbindungsstelle ist insbesondere der Schnittansicht aus Fig. 5 entlang der Linie II-II in Figur 2 entnehmbar. Gemäß Fig. 5 wird die Federleiste 21 zur Befestigung entsprechend mittels der Befestigungsschraube 36 an einem Bauteil, gemäß Fig. 5 an der Zwischenwand 34, der Seitenwand (in Fig. 5 an der Seitenwand 4) befestigt. In den Befestigungsbereichen der Federleiste 19 weist die Federleiste 19 im Gegensatz zu den Bereichen mit den Feststellelementen 20 auf Höhe der Objektlagerräume somit kein blattfederartiges Feststellelement 20 auf, das zur Feststellung des Objektes in einem Objektlagerraum ausgebildet ist, sondern im Wesentlichen allein den Befestigungsbereich 37.

Zur Freigabe des Objekts 24 wird die Stellleiste 19 motorgetrieben um ihre Schwenkachse in Pfeilrichtung C verschwenkt. Durch diese Schwenkbewegung schlägt die Stellleiste 19 mit einem Anschlagsbereich gegen die den Anschlagsbereich 29 der Federleiste 21 an und drückt den Anschlagbereich 29 in Pfeilrichtung D. Dadurch wird die Federleiste 21 um ihren Drehpunkt, der durch die Befestigungsschraube 36 im Befestigungsbereich 37 festgelegt wird, in die "Freigabe"-Position des Feststellelementes 20 verschwenkt. Jedes der Feststellelemente 20 ist somit einen zweiarmiger Hebel mit dem Feststellhebel 40, der das Objekt 24 zu dessen Feststellung kontaktiert und in der Objektlagerstelle festlegt, und dem Steuerhebel 41, an den die Stellleiste 19 gegen die Federleiste 21 anschlägt. Durch die Schwenkbewegung der Stellleiste 19 wird somit das Feststellelement 20 um seinen Drehpunkt und entgegen der auf den Feststellhebel 40 in "Feststell"-Position wirkenden Druckfeder 42 verschwenkt.

Die Bewegungsabläufe des Feststellhebels 40, des Steuerhebels 41, der Stellleiste 19 und der Druckfeder 42 zueinander sind in den Figuren 3b und 4b durch die Pfeile C-F näher veranschaulicht. Dabei betreffen die Pfeile C-E die Bewegungsrichtungen der Stellleiste 19 (Pfeil C), des Steuerhebels 41 (Pfeil D) und des Feststellhebels 40 (Pfeil E) von der "Feststell"-Position gemäß Fig. 3b in die "Freigabe"-Position gemäß Fig. 4b. Entsprechend betreffen die Pfeile C', D' und E' die Bewegungsrichtungen der betreffenden Elemente von der "Freigabe"-Position in die "Feststell"-Position. Pfeil F gibt in den Figuren 3b und 4b die Richtung der von der Druckfeder 42 auf den Feststellhebel 40 des Feststellelementes 20 ausgeübten Federkraft wieder. Ein wesentliches Merkmale der Anordnung ist somit, dass die Stellleiste 19 durch eine Schwenkbewegung in Richtung C letztendlich den Feststellhebel 40 in Richtung E entgegen der Federspannung der Druckfeder 42 verschwenkt und somit die Feststellvorrichtung 15 in die "Freigabe"-Position schaltet. Ferner drückt die Druckfeder 42 in Richtung F auf den Feststellhebel 40 und stellt somit, sobald die Stellleiste 19 in Richtung der Ausgangsposition gemäß Pfeil C' aus Fig. 3b geschwenkt wird, den Feststellhebel 40 in Richtung C' wieder in die "Feststell-Position". Die Stellleiste 19 steuert die Bewegung des Feststellhebels 40 in die "Feststell"-Position somit indirekt.

## Patentansprüche

1. Objektlagervorrichtung (1) mit Schüttelfünktion mit einer senkrecht angeordneten Lagerkassette (2), die mehrere übereinander angeordnete Objektlagerstellen aufweist, und mit einer über eine erste Antriebsverbindung mit der Lagerkassette funktional verbundene Antriebseinrichtung, umfassend ein Antriebselement zum Durchführen einer Schüttelbewegung der Lagerkassette (2),
**dadurch gekennzeichnet,**
**dass** die Antriebseinrichtung ferner über eine zweite Antriebsverbindung mit der Lagerkassette (2) funktional verbunden ist, wobei die erste Antriebsverbindung mit einem Bodenbereich und die zweiten Antriebsverbindung mit einem Kopfbereich der Lagerkassette (2) verbunden ist, und dass eine Steuereinrichtung vorhanden ist, die zur Synchronisation der beiden Antriebsverbindungen ausgebildet ist.

2. Öbjektlagervorrichtung (1) nach dem vorhergehenden Anspruch,
**dadurch gekennzeichnet,**
**dass** die erste und die zweite Antriebsverbindung einander gegenüberliegen und die erste Antriebsverbindung von unten und die zweite Antriebsverbindung von oben mit der Lagerkassette (2) funktional verbunden ist.

3. Objektlagervorrichtung (1) nach einem der vorhergehenden Ansprüche,
**dadurch gekennzeichnet,**
**dass** das Antriebselement ein elektromagnetischer Antrieb ist.

4. Objektlagervorrichtung (1) nach einem der vorhergehenden Ansprüche,
**dadurch gekennzeichnet,**
**dass** die Antriebseinrichtung zur Durchführung unterschiedlicher Schüttelbewegungen und insbesondere zur Durchführung von Orbital-, Linear- und Diagonalschüttelbewegungen ausgebildet ist.

5. Objektlagervorrichtung (1) nach einem der vorhergehenden Ansprüche,
**dadurch gekennzeichnet,**
**dass** die Antriebseinrichtung ein erstes (11) und ein zweites (12) Antriebselement aufweist, wobei das erste Antriebselement (11) über die erste Antriebsverbindung und das zweite Antriebselement (12) über die zweite Antriebsverbindung mit der Lagerkassette (2) verbunden ist, und wobei die Steuereinheit zur Synchronisation der durch das erste (11) und das zweite (12) Antriebselement durchgeführten Schüttelbewegung ausgebildet ist.

6. Objektlagervorrichtung (1) nach Anspruch 5,
**dadurch gekennzeichnet,**
**dass** das erste (11) und das zweite (12) Antriebselement baugleich sind.

7. Objektlagervorrichtung (1) nach einem der vorhergehenden Ansprüche,
**dadurch gekennzeichnet,**
**dass** die Antriebeinrichtung und insbesondere das erste (11) und das zweite (12) Antriebselement der Antriebseinrichtung feuchtigkeitsdicht ausgebildet sind.

8. Objektlagervorrichtung (1) nach einem der vorhergehenden Ansprüche,
**dadurch gekennzeichnet,**
**dass** die Objektlagervorrichtung (1) wenigstens zwei Lagerkassetten (2) aufweist, wobei jede der Lagerkassetten (2) eine erste Antriebsverbindung und eine zweite Antriebsverbindung aufweist.

9. Objektlagervorrichtung (1) nach Anspruch 8,
**dadurch gekennzeichnet,**
**dass** die Steuereinheit zur getrennten Steuerung der wenigstens zwei Lagerkassetten (2) ausgebildet ist.

10. Objektlagervorrichtung (1) nach einem der vorhergehenden Ansprüche,
**dadurch gekennzeichnet,**
**dass** eine Feststellvorrichtung (15) mit einem Feststellelement (20) zum Feststellen eines Objektes (24) und insbesondere eines plattenförmigen Probenträgers in einer der Objektlagerstellen und mit einer Stellvorrichtung (19) vorhanden ist, wobei die Stellvorrichtung (19) zum Verstellen des Feststellelementes (20) zwischen einer "Feststell"-Position und einer "Freigabe"-Position ausgebildet ist, und wobei das Objekt (24) in der "Feststell"-Position in einer der Objektlagerstellen festgestellt ist und in der "Freigabe"-Position aus dieser Objektlagerstelle entnehmbar ist.

11. Objektlagervorrichtung (1) nach Anspruch 10,
**dadurch gekennzeichnet,**
**dass** über die Stellvorrichtung (19) Feststellelemente (20) in mehreren Objektlagerstellen und insbesondere in allen Objektlagerstellen einer Lagerkassette (2) kombiniert zwischen der "Feststell"-Position und der "Freigabe"-Position verstellbar sind.

12. Objektlagervorrichtung (1) nach Anspruch 11,
**dadurch gekennzeichnet,**
**dass** die Stellvorrichtung (19) eine vertikal angeordnete Stellleiste (19) zur kombinierten Schaltung aller Feststellelemente (20) der Lagerkassette (2) zwischen der "Feststell"-Position und der "Freigabe"-Position aufweist.

13. Objektlagervorrichtung (1) nach einem der Ansprüche 10 bis 12,
**dadurch gekennzeichnet,**
**dass** das Feststellelement (20) einen zweiarmigen Hebel mit einem Steuerhebel (41) und mit einem Feststellhebel (40) umfasst, wobei der Steuerhebel (41) zur Schaltung des Feststellelementes (20) von der "Feststell"-Position in die "Freigabe"-Position mit der Stellvorrichtung (19) und insbesondere mit der Stellleiste (19) in Kontakt stehend ausgebildet ist, und wobei der Feststellhebel (40) in der Weise ausgebildet ist, dass er zur Feststellung des Objekts (24) in der Objektlagerstelle zumindest mit einem Teilbereich seitlich gegen das Objekt (24) drückt.

14. Objektlagervorrichtung (1) nach Anspruch 13,
**dadurch gekennzeichnet,**
**dass** der zweiarmige Hebel eine Blattfeder (21) ist.

15. Objektlagervorrichtung (1) nach einem der Ansprüche 13 oder 14,
**dadurch gekennzeichnet,**
**dass** der Feststellhebel (40) in der "Feststell"-Position das in die Objektlagerstelle eingebrachte Objekt (24) im Bereich der Objektvorderkante zur Verhinderung eines Herausrutschens des Objektes (24) aus der Objektlagerstelle umgreift.

16. Objektlagervorrichtung (1) nach einem der Ansprüche 13 bis 15,
**dadurch gekennzeichnet,**
**dass** der Feststellhebel (40) in der Weise federbeaufschlagt ist, dass die Federbeaufschlagung in die "Feststell"-Position des Feststellhebels (40) wirkt.

17. Objektlagervorrichtung (1) nach einem der Ansprüche 10 bis 16,
**dadurch gekennzeichnet,**
**dass** die Feststellvorrichtung (15) motorgetrieben ist.

18. Objektlagervorrichtung (1) nach einem der Ansprüche 10 bis 17,
**dadurch gekennzeichnet,**
**dass** die Feststellvorrichtung (15) ein selbsthemmendes Getriebe aufweist.

19. Objektlagervorrichtung (1) nach einem der Ansprüche 10 bis 18,
**dadurch gekennzeichnet,**
**dass** sie eine Arretiervorrichtung zum Arretieren der Lagerkassette (2) in einer definierten Be- und Entladeposition aufweist, wobei die Arretiervorrichtung in der Weise ausgebildet ist, dass sie die Lagerkassette (2) solange arretiert, solange sich das Feststellelement (20) in der "Freigabe"-Position befindet.

20. Klimaschrank, besonders Inkubator und insbesondere automatisierter Inkubator, mit einer Objektlagervorrichtung (1) mit Schüttelfunktion,
**dadurch gekennzeichnet,**
**dass** die Objektlagervorrichtung (1) gemäß einem der Ansprüche 1 bis 19 ausgebildet ist.

## Claims

1. An object storage device (1) having shaking function having a vertically situated storage cassette (2), which has multiple object storage points situated one on top of another, and having a drive unit functionally connected to the storage cassette via a first drive connection, comprising a drive element for performing a shaking movement of the storage cassette (2),
**characterized in that** the drive unit is also functionally connected to the storage cassette (2) via a second drive connection, the first drive connection being connected to a floor area and the second drive connection being connected to a head area of the storage cassette (2), and a control unit is provided, which is implemented to synchronize the two drive connections.

2. The object storage device (1) according to the preceding claim,
**characterized in that** the first and second drive connections are opposite one another and the first drive connection is functionally connected from below and the second drive connection is functionally connected from above to the storage cassette (2).

3. The object storage device (1) according to one of the preceding claims,
**characterized in that** the drive element is an electromagnetic drive.

4. The object storage device (1) according to one of the preceding claims,
**characterized in that** the drive unit is implemented to perform various shaking movements and in particular to perform orbital, linear, and diagonal shaking movements.

5. The object storage device (1) according to one of the preceding claims,
**characterized in that** the drive unit has a first drive element (11) and a second drive element (12), the first drive element (11) being connected to the storage cassette (2) via the first drive connection and the second drive element (12) being connected via the second drive connection, and the control unit being implemented to synchronize the shaking movement performed by the first drive element (11) and the second drive element (12).

6. The object storage device (1) according to Claim 5,
**characterized in that** the first drive element (11) and the second drive element (12) have identical constructions.

7. The object storage device (1) according to one of the preceding claims,
**characterized in that** the drive unit and in particular the first drive element (11) and the second drive element (12) of the drive unit are implemented as moisture-tight.

8. The object storage device (1) according to one of the preceding claims,
**characterized in that** the object storage device (1) has at least two storage cassettes (2), each of the storage cassettes (2) having a first drive connection and a second drive connection.

9. The object storage device (1) according to Claim 8,
**characterized in that** the control unit is implemented for separate control of the at least two storage cassettes (2).

10. The object storage device (1) according to one of the preceding claims,
**characterized in that** a fixing device (15) is provided having a fixing element (20) for fixing an object (24) and in particular a plate-shaped sample carrier in one of the object storage points and having an actuating device (19), the actuating device (19) being implemented to adjust the fixing element (20) between a "fix" position and a "release" position, and the object (24) being fixed in one of the object storage points in the "fix" position and being removable from this object storage point in the "release" position.

11. The object storage device (1) according to Claim 10,
**characterized in that** fixing elements (20) in multiple object storage points and in particular in all object storage points of the storage cassette (2) are adjustable in a combined way between the "fix" position and the "release" position via the actuating device (19).

12. The object storage device (1) according to Claim 11,
**characterized in that** the actuating device (19) has a vertically situated actuating strip (19) for the combined switching of all fixing elements (20) of the storage cassette (2) between the "fix" position and the "release" position.

13. The object storage device (1) according to one of Claims 10 through 12,
**characterized in that** the fixing element (20) comprises a two-arm lever having a control lever (41) and a fixing lever (40), the control lever (41) for switching the fixing element (20) from the "fix" position into the "release" position being implemented as in contact with the actuating device (19) and in particular with the actuating strip (19), and the fixing lever (40) being implemented in such a way that it presses with at least a partial area laterally against the object (24) to fix the object (24) in the object storage point.

14. The object storage device (1) according to Claim 13,
**characterized in that** the two-arm lever is a leaf spring (21).

15. The object storage device (1) according to one of Claim 13 or 14,
**characterized in that**, in the "fix" position, the fixing lever (40) encloses the object (24) introduced into the object storage point in the area of the object front edge to prevent the object (24) from slipping out of the object storage point.

16. The object storage device (1) according to one of Claim 13 to 15,
**characterized in that** the fixing lever (40) is spring-loaded in such a way that the spring loading acts in the "fix" position of the fixing lever (40).

17. The object storage device (1) according to one of Claims 10 through 16,
**characterized in that** the fixing device (15) is motor-driven.

18. The object storage device (1) according to one of Claims 10 through 17,
**characterized in that** the fixing device (15) has a self-locking gear.

19. The object storage device (1) according to one of Claims 10 through 18,
**characterized in that** it has a locking device for locking the storage cassette (2) in a defined loading and unloading position, the locking device being implemented in such a way that it locks the storage cassette (2) as long as the fixing element (20) is located in the "release" position.

20. A climatic cabinet, especially an incubator and particularly an automated incubator, having an object storage device (1) having shaking function,
**characterized in that** the object storage device (1) is implemented according to one of Claims 1 through 19.

## Revendications

1. Dispositif porte-objet (1) avec fonction d'agitation, avec une cassette de support (2) disposée verticalement qui présente plusieurs emplacements d'objet superposés, et avec un dispositif d'entraînement relié à la cassette de support par une première liaison d'entraînement et comprenant un élément d'entraînement pour l'exécution d'un mouvement d'agitation de la cassette de support (2),
**caractérisé en ce que** le dispositif d'entraînement est en outre en liaison fonctionnelle avec la cassette de support (2) par l'intermédiaire d'une deuxième liaison d'entraînement, la première liaison d'entraînement étant reliée avec une partie de fond et la deuxième liaison d'entraînement avec une partie supérieure de la cassette de support (2), et **en ce qu'**il est prévu un dispositif de commande conçu pour la synchronisation des deux liaisons d'entraînement.

2. Dispositif porte-objet (1) selon la revendication précédente, **caractérisé en ce que** les première et deuxième liaisons d'entraînement se font face et la première liaison d'entraînement est en liaison fonctionnelle avec la cassette de support (2) par le bas et la deuxième liaison d'entraînement par le haut.

3. Dispositif porte-objet (1) selon l'une des revendications précédentes, **caractérisé en ce que** l'élément d'entraînement est un entraînement électromagnétique.

4. Dispositif porte-objet (1) selon l'une des revendications précédentes, **caractérisé en ce que** le dispositif d'entraînement est conçu pour exécuter différents mouvements d'agitation, et en particulier pour exécuter des mouvements d'agitation orbitaux, linéaires et en diagonale.

5. Dispositif porte-objet (1) selon l'une des revendications précédentes, **caractérisé en ce que** le dispositif d'entraînement présente un premier élément d'entraînement (11) et un deuxième (12), le premier élément d'entraînement (11) étant relié à la cassette de support (2) par la première liaison d'entraînement et le deuxième élément d'entraînement (12) par la deuxième liaison d'entraînement, et l'unité de commande étant conçue pour synchroniser le mouvement d'agitation exécuté par le premier élément d'entraînement (11) et le deuxième (12).

6. Dispositif porte-objet (1) selon la revendication 5, **caractérisé en ce que** le premier élément d'entraînement (11) et le deuxième (12) sont de construction identique.

7. Dispositif porte-objet (1) selon l'une des revendications précédentes, **caractérisé en ce que** le dispositif d'entraînement et en particulier le premier élément d'entraînement (11) et le deuxième (12) du dispositif d'entraînement sont conçus pour être étanches à l'humidité.

8. Dispositif porte-objet (1) selon l'une des revendications précédentes, **caractérisé en ce que** le dispositif porte-objet (1) présente au moins deux cassettes de support (2), chacune des cassettes de support (2) présentant une première liaison d'entraînement et une deuxième liaison d'entraînement.

9. Dispositif porte-objet (1) selon la revendication 8, **caractérisé en ce que** l'unité de commande est conçue pour commander séparément les au moins deux cassettes de support (2).

10. Dispositif porte-objet (1) selon l'une des revendications précédentes, **caractérisé en ce qu'**il est prévu un dispositif d'immobilisation (15) avec un élément d'immobilisation (20) pour immobiliser un objet (24), en particulier un support d'échantillon en forme de plaque, dans l'un des emplacements d'objet et avec un dispositif de réglage (19), lequel dispositif de réglage (19) est conçu pour déplacer l'élément d'immobilisation (20) entre une position d'immobilisation et une position de déblocage, et dans lequel l'objet (24) est immobilisé dans un des emplacements d'objet dans la position d'immobilisation et peut être retiré de cet emplacement d'objet dans la position de déblocage.

11. Dispositif porte-objet (1) selon la revendication 10, **caractérisé en ce que** le dispositif de réglage (19) peut déplacer de façon combinée des éléments d'immobilisation (20) dans plusieurs emplacements d'objet, et en particulier dans tous les emplacements d'objet d'une cassette de support (2), entre la position d'immobilisation et la position de déblocage.

12. Dispositif porte-objet (1) selon la revendication 11, **caractérisé en ce que** le dispositif de réglage (19) comporte une barre de réglage (19) verticale pour la commutation combinée de tous les éléments d'immobilisation (20) de la cassette de support (2) entre la position d'immobilisation et la position de déblocage.

13. Dispositif porte-objet (1) selon l'une des revendications 10 à 12, **caractérisé en ce que** l'élément d'immobilisation (20) comprend un levier à deux bras avec un levier de commande (41) et un levier d'immobilisation (40), le levier de commande (41) étant conçu en contact avec le dispositif de réglage (19) et en particulier avec la barre de réglage (19) pour faire passer l'élément d'immobilisation (20) de la position d'immobilisation à la position de déblocage et le levier d'immobilisation (40) étant conformé de telle sorte qu'afin d'immobiliser l'objet (24) dans l'emplacement d'objet, il appuie latéralement contre l'objet (24) avec au moins une zone partielle.

14. Dispositif porte-objet (1) selon la revendication 13, **caractérisé en ce que** le levier à deux bras est un ressort à lames (21).

15. Dispositif porte-objet (1) selon l'une des revendications 13 ou 14, **caractérisé en ce que** le levier d'immobilisation (40), dans la position d'immobilisation, entoure l'objet (24) introduit dans l'emplacement d'objet au niveau du bord avant de l'objet afin d'empêcher l'objet (24) de glisser hors de l'emplacement d'objet.

16. Dispositif porte-objet (1) selon l'une des revendications 13 à 15, **caractérisé en ce que** le levier d'immobilisation (40) est contraint par ressort de telle façon que la contrainte par ressort s'exerce dans la position d'immobilisation du levier d'immobilisation (40).

17. Dispositif porte-objet (1) selon l'une des revendications 10 à 16, **caractérisé en ce que** le dispositif d'immobilisation (15) est entraîné par un moteur.

18. Dispositif porte-objet (1) selon l'une des revendications 10 à 17, **caractérisé en ce que** le dispositif d'immobilisation (15) présente un engrenage autobloquant.

19. Dispositif porte-objet (1) selon l'une des revendications 10 à 18, **caractérisé en ce qu'**il comporte un dispositif de blocage pour le blocage de la cassette de support (2) dans une position de chargement et de déchargement définie, lequel dispositif de blocage est conçu de façon à bloquer la cassette de support (2) tant que l'élément d'immobilisation (20) se trouve dans la position de déblocage.

20. Armoire climatique, en particulier incubateur et notamment incubateur automatisé, avec un dispositif porte-objet (1) à fonction d'agitation, **caractérisée en ce que** le dispositif porte-objet (1) est conçu selon l'une des revendications 1 à 19.
